# EUROPÄISCHE PATENTSCHRIFT

(11) **EP 1 129 063 B1**
(45) Veröffentlichungstag und Bekanntmachung des Hinweises auf die Patenterteilung: **16.07.2003**
(21) Anmeldenummer: 99958010.3
(22) Anmeldetag: 08.11.1999
(51) Int. Cl.: C07C 209/66, C07C 209/26, C07C 209/16

(54) **VERFAHREN ZUR HERSTELLUNG VON CYCLOHEXANDIAMINEN**
METHOD FOR PRODUCING CYCLOHEXANEDIAMINES
PROCEDE DE PRODUCTION DE CYCLOHEXANDIAMINES

(30) Priorität: 12.11.1998 EP 98121482; 15.10.1999 US 159545 P
(43) Veröffentlichungstag der Anmeldung: 05.09.2001
(73) Patentinhaber: Lonza AG, 4052 Basel (CH)
(72) Erfinder: BAIKER, Alfons, CH-8152 Opfikon (CH); FISCHER, Achim, D-63517 Rodenbach (DE); MALLAT, Tamas, CH-8057 Zürich (CH)
(86) Internationale Anmeldenummer: EP9908553
(87) Internationale Veröffentlichungsnummer: WO00029367

(56) Entgegenhaltungen:
- US-A- 5 773 657
- US-A- 5 789 620
- FISCHER, A. ET AL: "Synthesis of 1,4-Diaminocyclohexane in Supercritical Ammonia" JOURNAL OF CATALYSIS , [Online] Bd. 182, Nr. 2, 1999, Seiten 289-291, XP002129431 Retrieved from the Internet: <URL:http://www.apnet.com/www/journal/ca.h tml> [retrieved on 2000-02-01]

## Beschreibung

Die Erfindung betrifft ein Verfahren zur Herstellung von 1,*n*-Cyclohexandiaminen, wobei *n* die Werte 2, 3 und 4 annehmen kann. Diese Verbindungen besitzen die allgemeine Formel 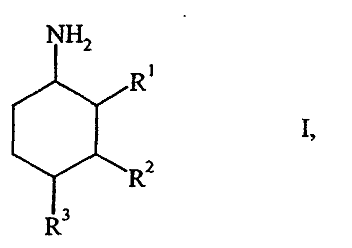 worin einer der Reste R¹ bis R³ eine Aminogruppe ist und die beiden übrigen Wasserstoff bedeuten.

1,*n*-Cyclohexandiamine, insbesondere das 1,4-Cyclohexandiamin (R³ =NH₂), sind potentiell vielseitig verwendbare Verbindungen. Von Interesse ist insbesondere ihre Verwendung als Bausteine für Polymere, zum Beispiel als Aminokomponente für die Herstellung von Polyamiden, Polyimiden oder Polyharnstoffen oder als Ausgangsmaterial für die Herstellung von Diisocyanaten zur Herstellung von Polyurethanen. Weiterhin sind diese Verbindungen zweizähnige Liganden, die mit verschiedenen Übergangsmetallen Komplexe bilden, wovon einige pharmakologische Wirkung besitzen, beispielsweise als Cytostatika.

Bisher bekannte Verfahren zur Herstellung von 1,*n*-Cyclohexandiaminen beruhen auf der Hydrierung der entsprechenden Phenylendiamine. Diese sind aber relativ teuer, sehr toxisch und, insbesondere das *para*-Isomere, empfindlich gegen Oxidation. Es sind auch Verfahren zur direkten Hydrierung der entsprechenden Nitroaniline bekannt, welche aber ebenfalls sehr toxisch sind.

Aufgabe der vorliegenden Erfindung war daher, ein für die Durchführung in technischem Massstab geeignetes Verfahren zur Herstellung von 1,*n*-Cyclohexandiaminen bereitzustellen, welches von leicht zugänglichen und relativ ungefährlichen Verbindungen ausgeht.

Erfindungsgemäss wird diese Aufgabe durch das Verfahren nach Patentanspruch 1 gelöst.

Es wurde gefunden, dass 1,*n*-Cyclohexandiole der allgemeinen Formel 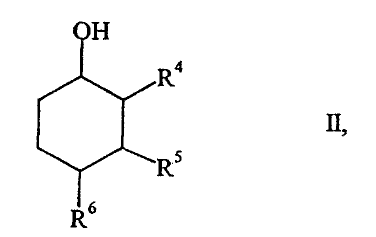 worin einer der Reste R⁴ bis R⁶ eine Hydroxygruppe ist und die übrigen Wasserstoff bedeuten, durch Umsetzung mit Ammoniak in Gegenwart von Wasserstoff und eines Katalysators auf Basis von Cobalt, gegebenenfalls mit Zusatz von Eisen als Promotor, in einem einstufigen Prozess bei Reaktionsdrücken von 50 bis 300 bar und Temperaturen von 100 bis 350 °C in die gewünschten 1,*n*-Cyclohexandiamine übergeführt werden können. Die Mengenverhältnisse betragen zweckmässig 2-200 mol Ammoniak und 0,01-20 mol Wasserstoff auf 1 mol 1,*n*-Cyclohexandiol.
Bevorzugt sind Mengenverhältnisse von 10-100 mol, insbesondere 40-90 mol, Ammoniak und 2-10 mol Wasserstoff auf 1 mol 1,*n*-Cyclohexandiol.

Der Reaktionsdruck liegt vorzugsweise bei 100-300 bar.
Besonders bevorzugt sind Drücke von 125-200 bar und Temperaturen von 150-230 °C.

Der Metallanteil des Katalysators, gerechnet ohne einen gegebenenfalls vorhandenen Träger, beträgt vorzugsweise 90 bis 100 Gewichtsprozent.

Besonders bevorzugt sind Katalysatoren, die zusätzlich zum Cobalt 1 bis 60 Gewichtsprozent (bezogen auf den gesamten Metallgehalt) Eisen als Promotor enthalten. Es liegt auch im Rahmen der Erfindung, andere Promotoren wie beispielsweise Seltenerdmetalle einzusetzen.

Die erfindungsgemäss einsetzbaren Katalysatoren können beispielsweise dadurch hergestellt werden, dass man die Hydroxide, Oxidhydrate und/oder Hydroxycarbonate der aktiven Metalle bei pH 5-9 ausfällt, wäscht, trocknet und bei 200-500 °C, vorzugsweise 300-500 °C, in oxidierender Atmosphäre kalziniert.

Für die Durchführung der erfindungsgemässen Reaktion ist es von Vorteil, wenn auf der Katalysatoroberfläche basische und saure Zentren vorhanden sind, weil die Adsorption und Aktivierung des Ammoniaks und des als Zwischenprodukt auftretenden Aminoalkohols an diesen Zentren stattfinden kann. Stark basische oder stark saure Zentren sind allerdings von Nachteil, da sie die Selektivität der Reaktion herabsetzen und Nebenreaktionen wie Fragmentierung und Oligomerisierung begünstigen. Die sauren und/oder basischen Eigenschaften des Katalysators werden durch den pH-Wert bei der Fällung beeinflusst. Es ist daher wesentlich, die Fällung bei einem pH von 5 bis 9 durchzuführen.

Die Fällung kann beispielsweise durch Zugabe einer Base zu einer Lösung der Acetate, Nitrate oder Halogenide der Metallkomponente(n) erfolgen. Bevorzugte Basen sind Ammoniumcarbonat bzw. -carbamat oder Ammoniak.

Die Trocknung erfolgt vorzugsweise bei Temperaturen bis zu 150 °C, gegebenenfalls im Vakuum.

Der Katalysator wird vor Gebrauch vorteilhaft in reduzierender Atmosphäre (z. B. Wasserstoff) bei 200-400 °C aktiviert. In der aktiven Form liegt der Katalysator im wesentlichen in metallischer Form vor.

Der Katalysator kann auf einem Träger wie Siliciumdioxid (z. B. Kieselgur), Alumiumoxid oder Graphit aufgebracht sein. Zu diesem Zweck kann die Fällung in Gegenwart des Trägers durchgeführt werden.

Vorzugsweise wird das erfindungsgemässe Verfahren kontinuierlich durchgeführt.

Die Kontaktzeit (definiert als Quotient aus Katalysatormasse [g] und zugeführtem Molenstrom [mol/s] der Reaktionspartner bei kontinuierlicher Durchführung beträgt vorzugsweise 10.000 bis 100.000 g·s/mol.

Bei kontinuierlicher Reaktionsführung wird der nicht umgesetzte Alkohol und/oder das nicht umgesetzte Ammoniak vorzugsweise rezykliert. Hierdurch kann auch unter Bedingungen, die bei einmaliger Passage nur geringen Umsatz liefern, eine gute Produktausbeute erzielt werden.

Das Ammoniak kann dem Reaktor gasförmig oder flüssig zugeführt werden. Das den Reaktor verlassende Reaktionsgemisch wird vorteilhaft gekühlt, z. B. durch adiabatische Expansion, das Ammoniak abgetrennt und gegebenenfalls in den Prozess rückgeführt.

Die folgenden Beispiele verdeutlichen die Durchführung des erfindungsgemässen Verfahrens, ohne dass darin eine Einschränkung zu sehen ist.

### Beispiel 1

### Herstellung eines Co/Fe-Katalysators

Die Salze Co(NO₃)₂·6 H₂O und Fe(NO₃)₃·9 H₂O wurden im molaren Verhältnis 20: 1 und einer Gesamtkonzentration von 0,36 mol/1 in Wasser gelöst und bis zum Erreichen von pH 7 mit einer 20%igen wässrigen Lösung von handelsüblichem "Ammoniumcarbonat" (Gemisch aus Carbonat und Carbamat) versetzt, wobei Cobalt und Eisen als Hydroxycarbonate ausfielen.

Der Niederschlag wurde abfiltriert, sorgfältig mit Wasser gewaschen, bei 100 °C im Vakuum getrocknet und bei 400 °C für 2 h an der Luft kalziniert. Vor Gebrauch wurde der so erhaltene Katalysator 4 h bei 350 °C und 40 bar in Wasserstoff aktiviert. Der so erhaltene Katalysator hatte eine spezifische Oberfläche (nach BET) von 12 m²/g und einen mittleren Porendurchmesser von 43 nm. Gemäss Röntgenbeugungsdiagramm lag das Cobalt im wesentlichen in der β-Phase vor.

### Beispiel 2

### 1,4-Cyclohexandiamin

Die Reaktion wurde in einem Festbett-Rohrreaktor kontinuierlich durchgeführt. Der Reaktor bestand aus einem Rohr mit einem Innendurchmesser von 13 mm und einer Länge von 304 mm aus Inconel®-718. Er enthielt 8 g des Co/Fe-Katalysators aus Beispiel 1. Ein Reaktionsgemisch aus 1,4-Cyclohexandiol, Ammoniak und Wasserstoff im molaren Verhältnis 1:60:2 wurde von oben nach unten durch den Reaktor gepumpt. Die Reaktionstemperatur betrug 165 °C, der Druck 135 bar und die Kontaktzeit 40.000 g·s/mol. Es wurde ein Umsatz von 76% erzielt. Das Produktgemisch enthielt 1,4-Cyclohexandiamin und 4-Aminocyclohexanol entsprechend einer Selektivität von 55% und 42%.

### Beispiel 3

### 1,4-Cyclohexandiamin

Es wurde verfahren wie in Beispiel 2 beschrieben, jedoch betrug die Reaktionstemperatur 195 °C und die Kontaktzeit 20.000 g·s/mol. Der Umsatz war 99% und die Ausbeute an 1,4-Cyclohexandiamin 65%. Ausserdem wurden 6% 4-Aminocyclohexanol und 26% sonstige Produkte gefunden.

### Beispiel 4

### 1,4-Cyclohexandiamin

Es wurde verfahren wie in Beispiel 2 beschrieben, jedoch wurde das nicht umgesetzte Diol rezykliert. Der Umsatz war 95%, die Ausbeute an 1,4-Cyclohexandiamin 51%. Ausserdem wurden 38% 4-Aminocyclohexanol und 5% sonstige Produkte gefunden.

## Patentansprüche

1. Verfahren zur Herstellung von 1,*n*-Cyclohexandiaminen der allgemeinen Formel 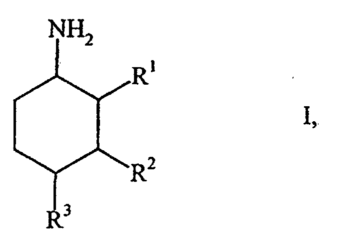 worin einer der Reste R¹ bis R³ eine Aminogruppe ist und die beiden übrigen Wasserstoff bedeuten, **dadurch gekennzeichnet, dass** ein entsprechendes 1,*n*-Cyclohexandiol der allgemeinen Formel 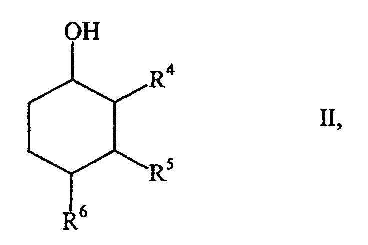 worin einer der Reste R⁴ bis R⁶ eine Hydroxygruppe ist und die übrigen Wasserstoff bedeuten, in Gegenwart von Wasserstoff und eines bei pH 5 bis 9 gefällten Co enthaltenden Katalysators bei einer Temperatur von 100 bis 350 °C, einem Druck von 50 bis 300 bar und einem Verhältnis von 2 bis 200 mol Ammoniak und 0,01 bis 20 mol Wasserstoff pro mol 1,*n*-Cyclohexandiol mit Ammoniak umgesetzt wird.

2. Verfahren nach Anspruch 1, **dadurch gekennzeichnet, dass** der Metallanteil des Katalysators ohne Träger 90 bis 100 Gew.-% beträgt.

3. Verfahren nach Anspruch 1 oder 2, **dadurch gekennzeichnet, dass** der Katalysator als Promotor 1 bis 60 Gew.-% Fe enthält.

4. Verfahren nach einem der Ansprüche 1 bis 3, **dadurch gekennzeichnet, dass** der Katalysator durch Fällung der entsprechenden Hydroxide, Oxidhydrate und/oder basischen Carbonate und anschliessende Trocknung sowie Kalzination in oxidierender Atmosphäre bei 200 bis 500 °C hergestellt wurde.

5. Verfahren nach einem der Ansprüche 1 bis 4, **dadurch gekennzeichnet, dass** der Katalysator bei einer Temperatur von 200 bis 400 °C in reduzierender Atmosphäre aktiviert wurde.

6. Verfahren nach einem der Ansprüche 1 bis 5, **dadurch gekennzeichnet, dass** der Katalysator auf einem Träger aus Siliciumdioxid, Aluminiumoxid oder Graphit aufgebracht ist.

7. Verfahren nach einem der Ansprüche 1 bis 6, **dadurch gekennzeichnet, dass** es kontinuierlich durchgeführt wird.

8. Verfahren nach Anspruch 7, **dadurch gekennzeichnet, dass** die Kontaktzeit 10.000 bis 100.000 g·s/mol beträgt.

9. Verfahren nach Anspruch 7 oder 8, **dadurch gekennzeichnet, dass** das nicht umgesetzte 1,*n*-Cyclohexandiol und/oder das nicht umgesetzte Ammoniak rezykliert wird.

## Claims

1. Process for preparing 1,*n*-cyclohexanediamines of the general formula 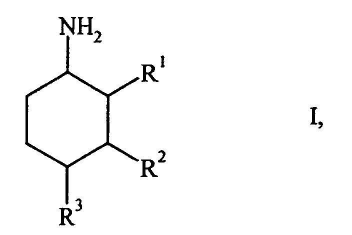 where one of the radicals R¹ to R³ is an amino group and the two others are hydrogen, **characterized in that** a corresponding 1,*n*-cyclohexanediol of the general formula 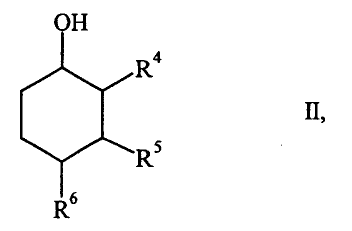 where one of the radicals R⁴ to R⁶ is a hydroxyl group and the others are hydrogen, is reacted with ammonia in the presence of hydrogen and of a Co-containing catalyst precipitated at pH 5-9 at a temperature of from 100 to 350 °C, at a pressure of from 50 to 300 bar and at a ratio of from 2 to 200 mol of ammonia and from 0.01 to 20 mol of hydrogen per mole of 1,*n*-cyclohexanediol.

2. Process according to Claim 1, **characterized in that** the metal fraction of the catalyst without support is from 90 to 100% by weight.

3. Process according to Claim 1 or 2, **characterized in that** the catalyst comprises from 1 to 60% by weight of Fe as promoter.

4. Process according to one of Claims 1 to 3, **characterized in that** the catalyst was prepared by precipitating the corresponding hydroxides, oxide hydrates and/or basic carbonates followed by drying, and also calcining in an oxidizing atmosphere at from 200 to 500 °C.

5. Process according to one of Claims 1 to 4, **characterized in that** the catalyst was activated at a temperature of from 200 to 400 °C in a reducing atmosphere.

6. Process according to one of Claims 1 to 5, **characterized in that** the catalyst has been applied to a support made from silicon dioxide, aluminium oxide or graphite.

7. Process according to one of Claims 1 to 6, **characterized in that** it is carried out continuously.

8. Process according to Claim 7, **characterized in that** the contact time is from 10,000 to 100,000 g·s/mol.

9. Process according to Claim 7 or 8, **characterized in that** the unreacted 1,*n*-cyclohexanediol and/or the unreacted ammonia is recycled.

## Revendications

1. Procédé pour la préparation de 1,*n*-cyclohexanediamines de formule générale 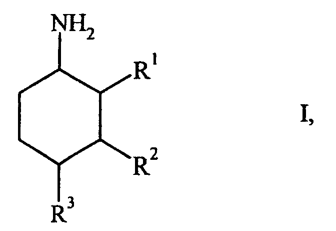 dans laquelle l'un des radicaux R1 à R3 est un groupe amino et les deux autres représentent des atomes d'hydrogène, **caractérisé en ce qu'**on fait réagir avec de l'ammoniac un 1,*n*-cyclohexanediol correspondant de formule générale 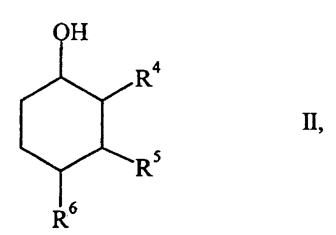 dans laquelle l'un des radicaux R4 et R6 est un groupe hydroxy et les autres représentent des atomes d'hydrogène, en présence d'hydrogène et d'un catalyseur contenant du Co, précipité à pH 5-9, à une température de 100 à 350 °C, sous une pression de 50 à 300 bars et à un rapport de 2 à 200 moles d'ammoniac et de 0,01 à 20 moles d'hydrogène par mole de 1,*n*-cyclohexanediol.

2. Procédé selon la revendication 1, **caractérisé en ce que** la teneur en métal du catalyseur sans support va de 90 à 100 % en poids.

3. Procédé selon la revendication 1 ou 2, **caractérisé en ce que** le catalyseur contient en tant qu'activateur 1 à 60 % en poids de Fe.

4. Procédé selon l'une quelconque des revendications 1 à 3, **caractérisé en ce que** le catalyseur a été préparé par préparation des hydroxydes, hydrates et/ou carbonates basiques correspondants et séchage subséquent ainsi que calcination en atmosphère oxydante, à 200-500 °C.

5. Procédé selon l'une quelconque des revendications 1 à 4, **caractérisé en ce que** le catalyseur a été activé à une température de 200 à 400 °C en atmosphère réductrice.

6. Procédé selon l'une quelconque des revendications 1 à 5, **caractérisé en ce que** le catalyseur est appliqué sur un support en dioxyde de silicium, oxyde d'aluminium ou graphite.

7. Procédé selon l'une quelconque des revendications 1 à 6, **caractérisé en ce qu'**il est mis en oeuvre en continu.

8. Procédé selon la revendication 7, **caractérisé en ce que** le temps de contact va de 10 000 à 100 000 g·s/mole.

9. Procédé selon la revendication 7 ou 8, **caractérisé en ce que** le 1,*n*-cyclohexanediol n'ayant pas réagi et/ou l'ammoniac n'ayant pas réagi est(sont) recyclé(s).
